# FASCICULE DE BREVET EUROPEEN

(11) **EP 3 817 704 B1**
(45) Date de publication et mention de la délivrance du brevet: **14.05.2025**
(21) Numéro de dépôt: 19759015.1
(22) Date de dépôt: 03.07.2019
(51) Int. Cl.: A61H 39/08, A61H 39/06, A61N 1/40, A61H 39/00, A61B 18/06, A61F 7/00

(54) **DISPOSITIF COMPRENANT UN OBJET AVEC UNE POINTE CHAUFFANTE ET BIOCOMPATIBLE**
VORRICHTUNG MIT EINEM GEGENSTAND MIT EINER BIOKOMPATIBLEN HEIZSPITZE
DEVICE COMPRISING AN OBJECT WITH A BIOCOMPATIBLE HEATING TIP

(30) Priorité: 03.07.2018 FR 1856116
(43) Date de publication de la demande: 12.05.2021
(73) Titulaire: Axemox, 67000 Strasbourg (FR)
(72) Inventeur: KORSEC, Philippe, 67000 Strasbourg (FR); HUBE, Jean-Michel, 67000 Strasbourg (FR); MATTERN, Antoine, 67000 Strasbourg (FR); MEYER, Robin, 67000 Strasbourg (FR)
(74) Mandataire: Icosa
(86) Numéro de dépôt international: PCT/FR2019/051644
(87) Numéro de publication internationale: WO 2020/008143

(56) Documents cités:
- WO-A1-2016/032109
- CN-A- 103 654 946
- CN-U- 205 672 215
- KR-A- 20090 011 157
- US-A1- 2010 249 770
- US-A1- 2012 157 749

## Description

### DOMAINE DE L'INVENTION

La présente invention concerne un dispositif comprenant un objet avec une pointe chauffante, tranchante ou pointue, biocompatible utilisable dans les traitements traditionnels tels que l'acupuncture ou la moxibustion, voire dans tout tableau physiopathologique indiquant la moxibustion en tant que moyen thérapeutique en médecine, et en particulier en médecine traditionnelle chinoise.

La présente invention peut notamment être utilisée dans le traitement cosmétique des ridules, des cernes et de la cellulite superficielle ou encore dans le traitement des varices et/ou dans le traitement des syndromes reliés à des tableaux de spondylalgies et des douleurs reliées à ces différents tableaux, en particulier des lombalgies ou des cervicalgies chroniques et de leurs douleurs projetées.

### ÉTAT DE LA TECHNIQUE

L'acupuncture ou la moxibustion sont des techniques traditionnelles thérapeutiques liées à la tradition médicale chinoise. L'acupuncture consiste en une stimulation de zones précises de la peau (derme, épiderme, hypoderme) dits « points d'acupuncture » au moyen d'aiguilles plus ou moins fines. La moxibustion consiste en une stimulation par la chaleur des points d'acupuncture. Ces techniques peuvent être utilisées pour de nombreuses applications et notamment le traitement ou l'accompagnement de nombreux troubles telles que les troubles articulaires, les dermatoses, les douleurs aigües ou chroniques, etc.

L'utilisation de ces techniques traditionnelles présente parfois des inconvénients. En particulier, dans le cas de la moxibustion, il y a, d'une part, un risque élevé de brûlure de la peau, et d'autre part, un risque d'occasionner un dégagement important et gênant de fumées pouvant être toxiques. De plus, cette technique ne permet pas de contrôler de manière précise la température de la zone chauffée.

Enfin, la moxibustion ne peut pas être utilisée dans un espace confiné (par exemple dans un cabinet ou une station orbitale) en raison du dégagement de fumée sauf à disposer de hotte d'aspiration de fumée ou de moxa ne dégageant pas de fumées pouvant être toxiques.

Il existe donc un besoin de faire évoluer cette technique pour garantir une utilisation sécurisée, fiable et pratique.

Il a été proposé par le document WO95/20935, une aiguille d'acupuncture chauffante comprenant une source de chaleur externe et un moyen de chauffage sous forme de conducteur thermique intégré dans l'aiguille sur toute la longueur de celle-ci. Par une application de chaleur grâce à un système de chauffage au niveau de la partie supérieure de l'aiguille, il en résulte un transfert par conduction de chaleur, le long du système de chauffage jusqu'à la pointe de l'aiguille et un rayonnement de cette chaleur à travers l'aiguille sur toute la longueur de l'aiguille.

Le premier inconvénient de cette technique est qu'elle impose une aiguille comprenant plusieurs couches successives et est donc difficile à fabriquer. Le second inconvénient est, que les différentes couches réalisées pour former le moyen de chauffage peuvent, de par l'application répétée de chaleur ou d'électricité, se détériorer rapidement avec risque de court-circuit contre-indiquant l'utilisation. Enfin, cette technique ne permet pas un contrôle précis de la température à la pointe de l'aiguille.

Le document CN 20276168 décrit un système de moxibustion où la température est contrôlée par induction. Néanmoins, ce document ne concerne que la moxibustion « externe » c'est-à-dire à la surface de la peau. Par conséquent, il n'y a aucun moyen de contrôler la température appliquée à l'intérieur du corps de manière précise.

La demande de brevet US2010/0249770 décrit un système de résection chirurgicale comprenant des aiguilles chauffées.

La demande de brevet internationale WO2016/032109 décrit un dispositif d'acupuncture comprenant des aiguilles d'acupuncture chauffante avec toutes les caractéristiques techniques du préambule de la revendication 1.

Ainsi, il existe le besoin d'un nouveau dispositif chauffant permettant d'améliorer les techniques traditionnelles.

### RÉSUMÉ

La présente invention concerne un dispositif selon la revendication 1. Les aspects subsidiaires de l'invention sont fournis dans les revendications dépendantes.

La présente invention consiste en un dispositif comprenant un objet qui comprend une aiguille d'acupuncture chauffante, pointue, biocompatible, et qui comprend au moins un matériau ferromagnétique, un générateur de champ magnétique adapté pour diriger le champ magnétique du matériau ferromagnétique afin de produire une élévation de température dans l'aiguille d'acupuncture de l'objet, et un dispositif de contrôle de la température de l'aiguille d'acupuncture de l'objet. Ledit générateur de champ magnétique a la forme d'un galet cylindrique creux, destiné à être posé sur la peau, qui comprend un orifice traversé perpendiculairement par ledit objet Ledit générateur de champ magnétique a un diamètre allant de 20 mm à 80 mm, et une épaisseur allant de 0,5 mm à 25 mm.

Le dispositif selon l'invention permet d'obtenir un contrôle précis et sécurisé de la température à la pointe chauffante de l'objet au moyen du dispositif de contrôle de la température. Ainsi, la température de la pointe de l'objet peut être parfaitement calibrée à une température de consigne fixée par le praticien. De plus, le dispositif selon l'invention est facile à fabriquer et à mettre en œuvre.

Dans un mode de réalisation, ledit générateur de champs magnétique est constitué d'une coque en matière isolante.

Dans un mode de réalisation, ledit générateur de champs magnétique est constitué d'une coque en matière conductrice, de préférence une coque en métal conducteur.

Dans un mode de réalisation, ledit orifice du générateur de champs magnétique a un diamètre allant de 0,1 mm à 10 mm, de préférence de 0,1 mm à 5 mm.

Dans un mode de réalisation, le matériau ferromagnétique est présent dans tout l'objet. Dans un mode de réalisation, l'objet est recouvert au moins en partie d'une couche de matériau isolant, de préférence est recouvert totalement d'une couche de matériau isolant à l'exception de la pointe chauffante.

Dans un mode de réalisation, l'objet comprend en outre un disque, isolant ou chauffant, destiné à être posé sur la peau qui comprend un orifice traversé perpendiculairement par l'objet.

Dans un mode de réalisation, le générateur de champ magnétique comprend en outre de la ferrite disposée de manière à diriger le champ magnétique selon l'axe longitudinal de l'objet.

Dans un mode de réalisation, le dispositif de contrôle de la température du dispositif de l'invention est relié au générateur de champ magnétique et comprend a) un dispositif d'affichage de la température de la pointe de l'objet - l'aiguille d'acupuncture -, b) des moyens pour déterminer une température de consigne, et c) un dispositif de contrôle du générateur de champ magnétique afin que la température de la pointe de l'objet - l'aiguille d'acupuncture - soit égale à la température de consigne.

Dans un mode de réalisation, le matériau ferromagnétique est choisi parmi les aciers, de préférence choisi parmi les aciers inoxydables ferritiques, les aciers inoxydables martensitiques, et les mélanges de ces aciers, plus préférentiellement choisi parmi l'acier inoxydable martensitique 420, l'acier inoxydable martensitique 416, l'acier inoxydable ferritique 430 et les mélanges de ces aciers, et en particulier le matériau ferromagnétique est l'acier inoxydable martensitique 420.

Dans un mode de réalisation, la pointe de l'objet - l'aiguille d'acupuncture - est recouverte au moins en partie d'un matériau biocompatible, de préférence le matériau biocompatible est choisi parmi le silicone, le polytétrafluoroéthylène (téflon), et un polymère hydrophile, et plus préférentiellement est le silicone.

### DÉFINITIONS

Dans la présente invention, les termes ci-dessous sont définis de la manière suivante :
- « **Biocompatibilité** » désigne la propriété d'un matériau provoquant peu ou pas de réponse immunitaire dans un organisme donné lorsque le matériau est en contact avec ledit organisme.
- « **Douleur projetée** » désigne une douleur ressentie à distance de la lésion causale. Ainsi, lorsque la douleur projetée est associée à une pathologie, la douleur n'est pas localisée à l'endroit de la pathologie mais est due cependant à cette pathologie.
- « **Environ** » placé devant un nombre signifie plus ou moins 10% de la valeur nominale de ce nombre.
- « **Ferrite** » désigne un mélange solide de carbone et de l'allotrope α du fer ou désigne l'allotrope α du fer.
- « **Ferromagnétique** » désigne la propriété d'un matériau de former un aimant permanent ou d'être attiré par un aimant.
- « **Matériau isolant** » désigne un matériau permettant d'isoler thermiquement ou électriquement, et de préférence thermiquement, une surface de contact d'une zone conductrice thermiquement ou électroniquement, et de préférence thermiquement.

### BRÈVE DESCRIPTION DES FIGURES

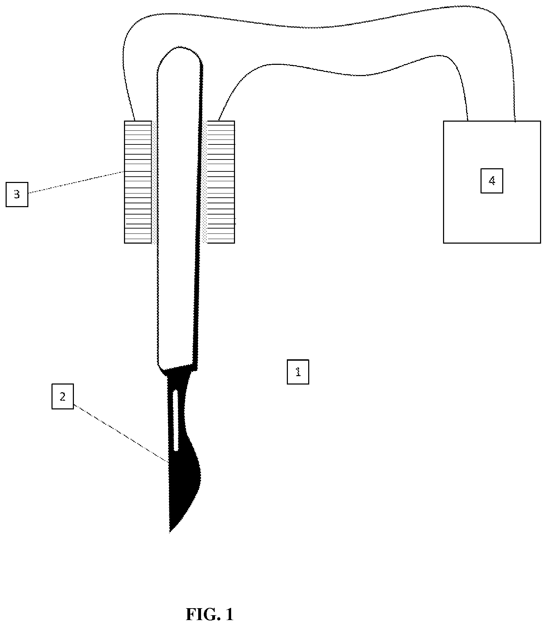

La **Figure 1** est un schéma d'un dispositif **1** permettant de comprendre le contexte de la présente invention : l'objet comprenant une pointe chauffante **2** est un scalpel.

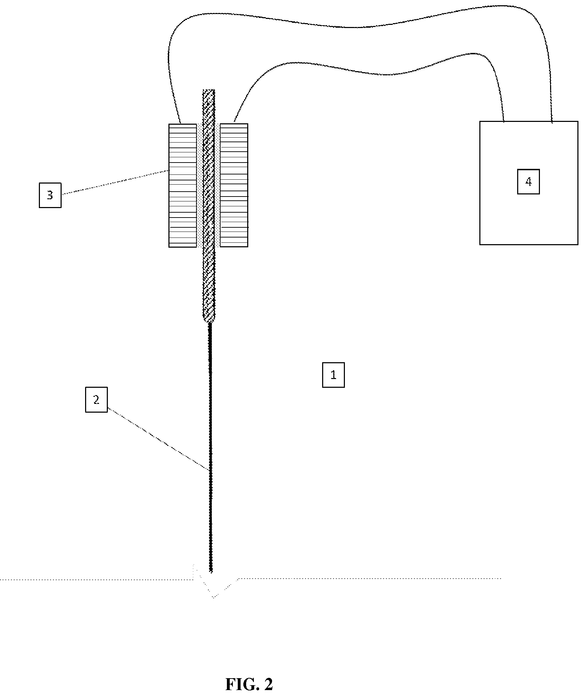

La **Figure 2** est un schéma d'un dispositif **1** permettant de comprendre le contexte de la présente invention : l'objet comprenant une pointe chauffante **2** est une aiguille d'acupuncture.

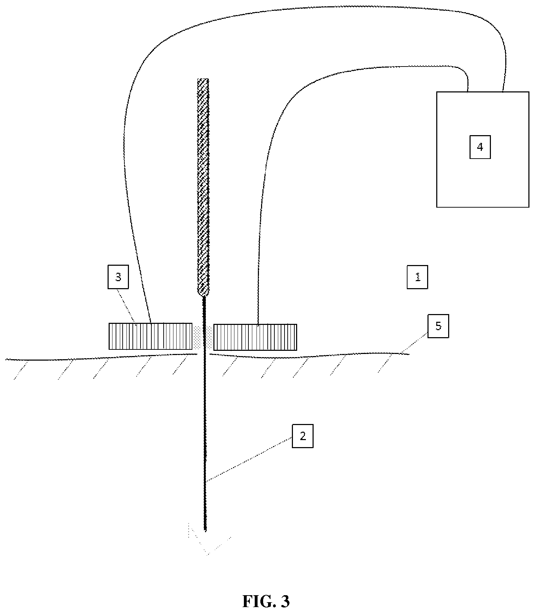

La **Figure 3** est un schéma d'un dispositif **1** selon un autre mode de réalisation de la présente invention. Dans ce mode de réalisation, l'objet comprenant une pointe chauffante **2** est une aiguille d'acupuncture. Dans ce mode de réalisation, le générateur de champs magnétique **3** à la forme d'un galet cylindrique posé sur la peau qui comprend un orifice traversé perpendiculairement par l'objet **2.**

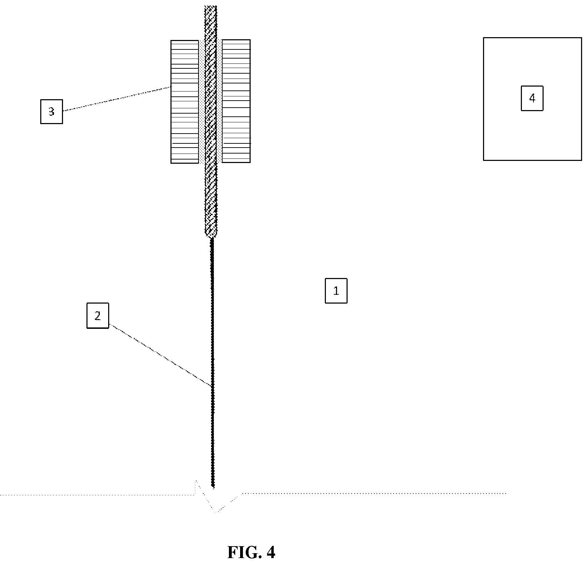

La **Figure 4** est un schéma d'un dispositif **1** permettant de comprendre le contexte de la présente invention : l'objet comprenant une pointe chauffante **2** est une aiguille d'acupuncture et la liaison entre l'objet comprenant une pointe chauffante et le dispositif de contrôle de la température **4** se fait au moyen d'une connexion sans fil comme par exemple une liaison Bluetooth ou wifi, ce qui permet notamment d'adjoindre au dispositif un traitement par ventouse.

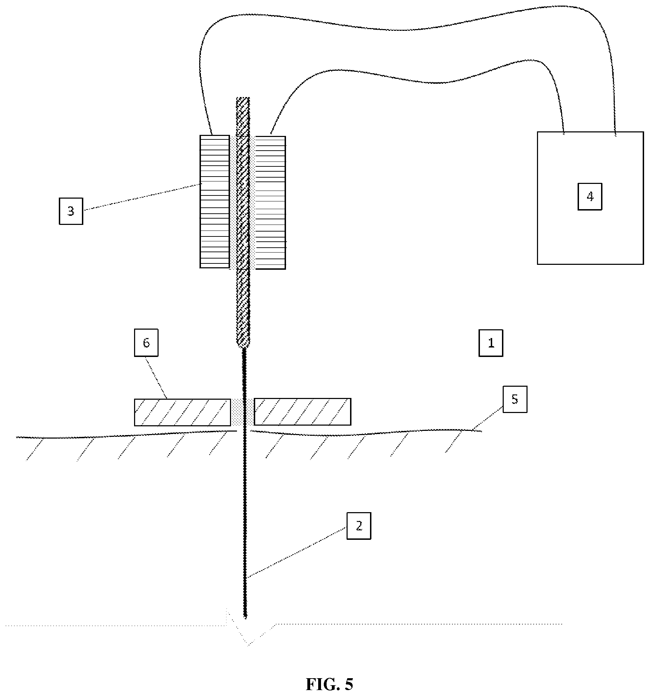

La **Figure 5** est un schéma d'un dispositif **1** permettant de comprendre le contexte de la présente invention : le dispositif comprend en outre un disque **6** posé sur la peau qui comprend un orifice traversé perpendiculairement par l'objet **2.**

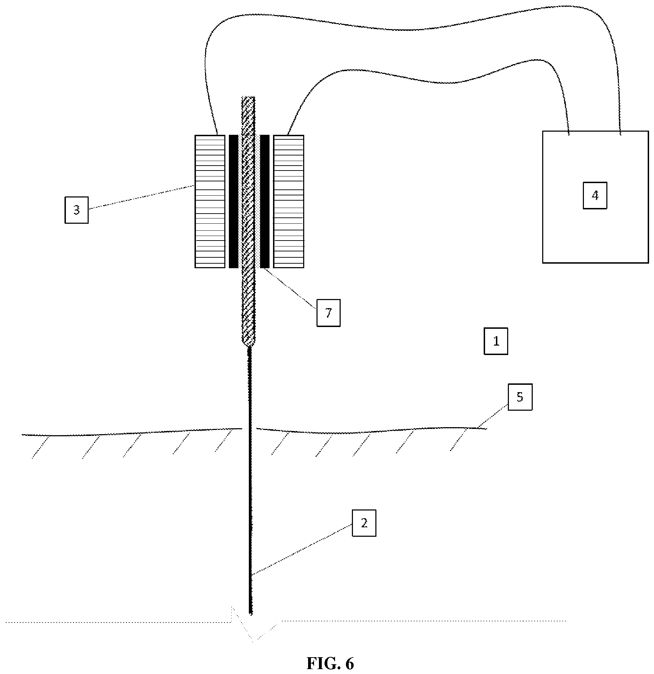

La **Figure 6** est un schéma d'un dispositif **1** permettant de comprendre le contexte de la présente invention : le générateur de champ magnétique **3** comprend en outre de la ferrite **7** disposée de manière à diriger le champ magnétique selon l'axe longitudinal de l'objet **2.**

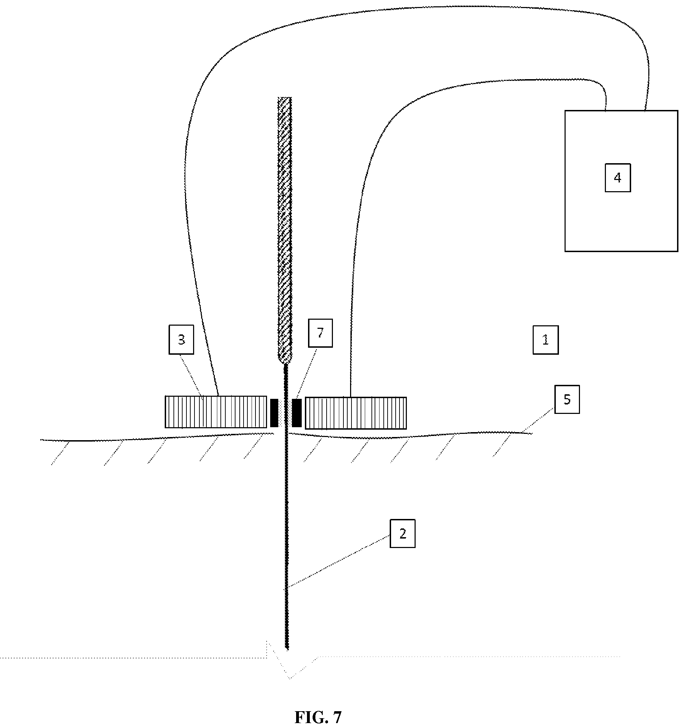

La **Figure 7** est un schéma d'un dispositif **1** selon un autre mode de réalisation de la présente invention. Dans ce mode de réalisation, le générateur de champ magnétique **3** à la forme d'un galet cylindrique posé sur la peau qui comprend un orifice traversé perpendiculairement par l'objet **2** et comprend en outre de la ferrite **7** disposée de manière à diriger le champ magnétique selon l'axe longitudinal de l'objet **2.**

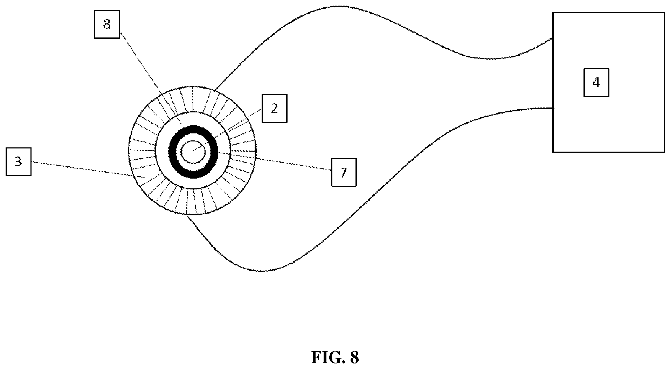

La **Figure 8** est une vue en coupe horizontale du dispositif **1** de la **Figure 6****.**

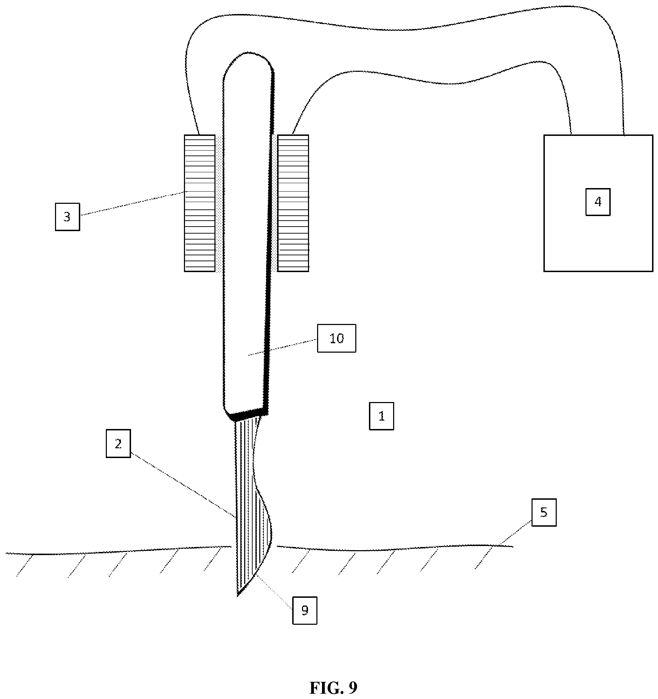

La **Figure 9** est un schéma d'un dispositif **1** permettant de comprendre le contexte de la présente invention : l'objet comprenant une pointe chauffante **2** est un scalpel et comprend dans tout l'objet **2** (et donc également dans la pointe chauffante) un matériau ferromagnétique **9.** L'objet **2** est recouvert au moins en partie d'une couche de matériau isolant **10,** de préférence est recouvert totalement d'une couche de matériau isolant **10** à l'exception de la pointe chauffante.

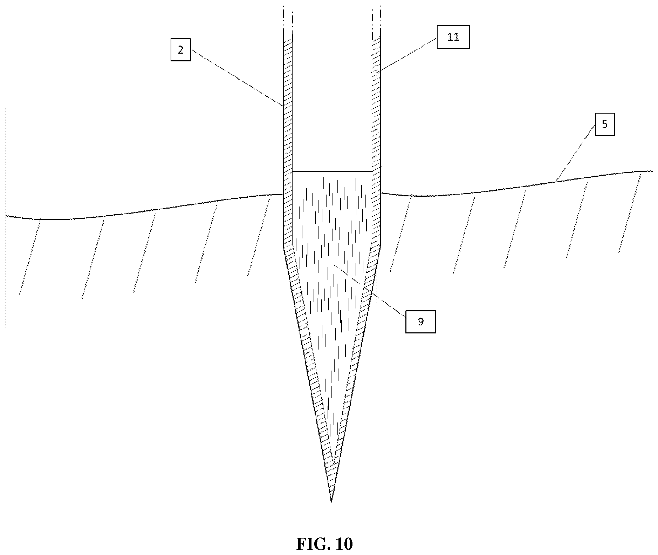

La **Figure 10** est un schéma de la pointe chauffante de l'objet **2** du dispositif **1** selon un autre mode de réalisation de la présente invention. Dans ce mode de réalisation, la pointe de l'objet **2** comprend un matériau ferromagnétique **9** et est recouverte d'un matériau biocompatible **11.**

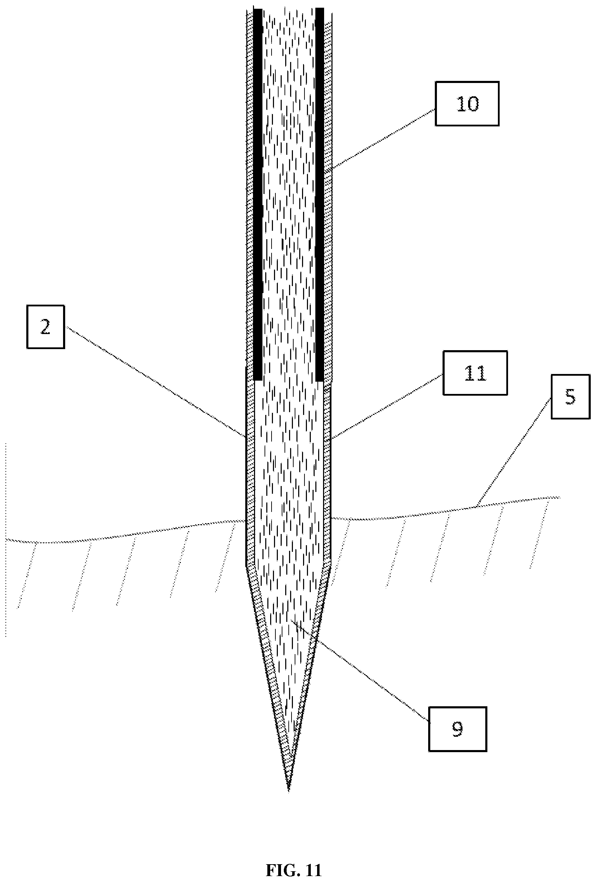

La **Figure 11** est un schéma de l'objet **2** du dispositif 1 selon un autre mode de réalisation de la présente invention. Dans ce mode de réalisation, le matériau ferromagnétique est compris dans tout l'objet **2.** L'objet **2** est recouvert au moins en partie d'une couche de matériau isolant **10,** de préférence est recouvert totalement d'une couche de matériau isolant **10** à l'exception de la pointe chauffante. L'objet **2** est recouvert totalement d'un matériau biocompatible **11.**

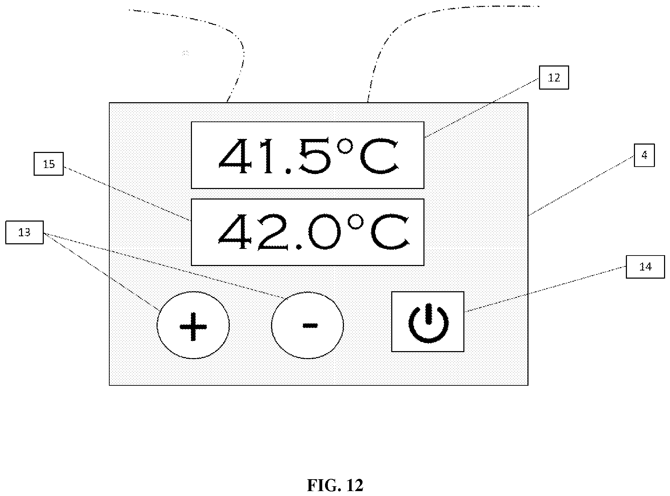

La **Figure 12** est un schéma du dispositif de contrôle de la température **4** du dispositif **1** selon un autre mode de réalisation de la présente invention. Dans ce mode de réalisation, le dispositif de contrôle de la température comprend un dispositif d'affichage de la température de la pointe de l'objet **12** et de la température de consigne **15,** des moyens pour déterminer une température de consigne **13,** et un interrupteur **14.**

### DESCRIPTION DÉTAILLÉE

La description suivante sera mieux comprise à la lecture des dessins. Dans le but d'illustrer l'invention, le dispositif est représenté dans des modes de réalisation préférés. Il doit être compris, cependant, que la présente demande n'est pas limitée aux arrangements, structures, caractéristiques, modes de réalisation et apparence précis indiqués. Les dessins ne sont pas dessinés à l'échelle et ne sont pas destinés à limiter la portée des revendications aux modes de réalisation représentés dans ces dessins. Par conséquent, il doit être compris que lorsque des caractéristiques mentionnées dans les revendications sont suivies par des références, lesdites références sont inclues uniquement en vue d'améliorer la compréhension des revendications et ne limitent en aucun cas la portée de ces revendications.

### Figures

Les Fig. 1, 2, 5, 6 et 9 montrent des dispositifs permettant de comprendre le contexte de l'invention, dans lesquels le dispositif **1** comprend un objet **2** comprenant une pointe chauffante, tranchante, biocompatible, telle qu'une lame de scalpel et qui comprend au moins un matériau ferromagnétique **9,** non représenté sur la Fig. 1. Le dispositif comprend également un générateur de champ magnétique **3** adapté pour diriger le champ magnétique du matériau ferromagnétique afin de produire une élévation de température dans la pointe chauffante de l'objet **2** et un dispositif de contrôle de la température **4** de la pointe chauffante de l'objet **2.** Le dispositif de contrôle de la température **4** agit sur le générateur de champ magnétique **3** en fournissant un courant. Ce courant va produire un champ magnétique. Le champ magnétique ainsi généré va venir orienter les particules du matériau ferromagnétique **9** (non représenté sur la Fig. 1) contenu dans l'objet **2,** de préférence dans tout l'objet **2,** et produire ainsi une augmentation de la température de tout l'intérieur de l'objet **2.** La température de la pointe de l'objet **2** peut être contrôlée au moyen par exemple d'un thermomètre ou d'un thermocouple (non représentés ici), le dispositif de contrôle de la température **4** modifiant ainsi le courant fournit au générateur de champ magnétique **3.** Comme représenté dans la Fig. 9, l'objet **2** est recouvert au moins en partie d'une couche de matériau isolant **10,** de préférence est recouvert totalement d'une couche de matériau isolant **10** à l'exception de la pointe chauffante. Ce matériau isolant peut être choisi parmi tous les matériaux isolants existants, et en particulier peut être choisi parmi les matières plastiques. Ce matériau isolant permet d'isoler thermiquement le reste de l'objet **2,** à part la pointe. Ainsi, seule la pointe de l'objet **2** est chaude et pas la totalité de l'objet **2.** La peau et les organes pouvant être en contact avec le reste de l'objet **2** ne sont pas chauffés.

Les Fig. 3, 7 et 8 montrent des modes de réalisation selon l'invention, dans lesquels le dispositif **1** comprend un objet **2** comprenant une pointe chauffante, pointue, biocompatible, telle qu'une aiguille d'acupuncture et qui comprend au moins un matériau ferromagnétique **9,** non représenté sur ces figures. Dans ces modes de réalisation, le dispositif comprend également un générateur de champ magnétique **3** adapté pour diriger le champ magnétique du matériau ferromagnétique afin de produire une élévation de température dans la pointe chauffante de l'objet **2** et un dispositif de contrôle de la température **4** de la pointe chauffante de l'objet **2.** Dans un mode de réalisation, le générateur de champs magnétique **3** n'est pas fixé à l'objet **2.** Le dispositif de contrôle de la température **4** agit sur le générateur de champ magnétique **3** en fournissant un courant. Ce courant va produire un champ magnétique. Le champ magnétique ainsi généré va venir orienter les particules du matériau ferromagnétique **9** (non représenté ici) contenu dans l'objet **2,** de préférence dans tout l'objet **2,** et produire ainsi une augmentation de la température de tout l'intérieur de l'objet **2.** La température de la pointe de l'objet **2** peut être contrôlée au moyen par exemple d'un thermomètre ou d'un thermocouple (non représentés ici), le dispositif de contrôle de la température **4** modifiant ainsi le courant fournit au générateur de champ magnétique **3.**

La Fig. 3 montre un mode de réalisation selon l'invention, dans lequel le générateur de champs magnétique **3** du dispositif **1** à la forme d'un galet cylindrique, destiné à être posé sur la peau **5** qui comprend un orifice traversé perpendiculairement par l'objet **2.** Dans un mode de réalisation, ledit galet est isolant, c'est-à-dire qu'il est généralement constitué d'une coque en matière isolante, telle qu'une coque en matière plastique, en verre ou en aluminium. Dans un autre mode de réalisation, ledit galet est chauffant, c'est-à-dire qu'il est généralement constitué d'une coque en matière conductrice, telle qu'un métal conducteur. Dans ce mode de réalisation, la matière conductrice constitutive de la coque dudit galet est de préférence la même que celle de la pointe chauffante de l'objet **2.** Lorsque ledit galet est chauffant, il peut être chauffé grâce au champ magnétique généré qui va venir orienter les particules du matériau ferromagnétique **9** (non représenté ici) contenu dans la coque dudit galet. L'utilisation dudit galet constitué d'une coque en matière isolante permet de ne pas chauffer la peau et de concentrer le chauffage uniquement sur la pointe chauffante de l'objet **2,** par exemple pour des traitements localisés. L'utilisation dudit galet constitué d'une coque en matière conductrice permet de chauffer la peau, par exemple pour des traitements non localisés.

La Fig. 4 montre un dispositif permettant de comprendre le contexte de l'invention, dans lequel l'objet **2** et le générateur de champ magnétique **3** sont reliés au dispositif de contrôle de la température **4** au moyen d'une connexion sans fil. Cette connexion sans fil peut être par exemple une connexion Bluetooth ou encore une connexion wifi ce qui permet notamment d'adjoindre au dispositif un traitement par ventouse.

La Fig. 5 montre un dispositif permettant de comprendre le contexte de l'invention, dans lequel le dispositif **1** comprend en plus un disque **6,** isolant ou chauffant, destiné à être posé sur la peau **5** qui comprend un orifice traversé perpendiculairement par l'objet **2.** Dans un mode de réalisation, le disque **6** est isolant, c'est-à-dire qu'il est généralement constitué d'une matière isolante, telle qu'une matière plastique ou du verre. Dans un autre mode de réalisation, le disque **6** est chauffant, c'est-à-dire qu'il est généralement constitué d'une matière conductrice, telle qu'un métal conducteur. Dans ce mode de réalisation, la matière conductrice constitutive du disque est de préférence la même que celle de la pointe chauffante de l'objet **2.** Lorsque le disque **6** est chauffant, il peut être chauffé de deux manières : soit indépendamment de la pointe chauffante de l'objet **2** par exemple par un système de chauffage soit par l'objet **2** par transfert de chaleur par conduction. L'utilisation supplémentaire du disque **6** isolant permet de ne pas chauffer la peau et de concentrer le chauffage uniquement sur la pointe chauffante de l'objet **2,** par exemple pour des traitements localisés. L'utilisation supplémentaire du disque **6** chauffant permet de chauffer la peau, par exemple pour des traitements non localisés.

La figure 6 montre un dispositif permettant de comprendre le contexte de l'invention et la figure 7 montre un mode de réalisation selon l'invention, dans lequel le générateur de champ magnétique **3** du dispositif **1** comprend en outre de la ferrite **7** disposée de manière à diriger le champ magnétique selon l'axe longitudinal de l'objet **2.** L'utilisation de la ferrite **7** présente l'avantage de diriger le champ magnétique produit par le générateur de champ magnétique **3** vers la pointe de l'objet chauffant **2.** Cela permet notamment de contrôler, de manière fine, la température atteinte par la pointe de l'objet chauffant **2.**

La Fig. 8 montre en vue de coupe horizontale le mode de réalisation tel que décrit dans les Fig 5 et 7. La ferrite **7** à la forme d'un cylindre et est disposée autour de l'objet **2.** Le générateur de champ magnétique **3** a également la forme d'un cylindre et est disposé autour de la ferrite **7.** Le support **8,** par exemple en matière plastique, sert à lier le générateur de champ magnétique **3** à la ferrite **7,** l'ensemble étant lié à l'objet **2,** ce lien n'étant pas représenté ici.

La Fig. 9 montre un dispositif permettant de comprendre le contexte de l'invention, dans lequel le matériau ferromagnétique **9** est localisé dans tout l'objet **2.** L'objet **2** est recouvert au moins en partie d'une couche de matériau isolant **10,** de préférence est recouvert totalement d'une couche de matériau isolant **10** à l'exception de la pointe chauffante. Ce matériau isolant peut être choisi parmi tous les matériaux isolants existants, et en particulier peut être choisi parmi les matières plastiques. Ce matériau isolant permet d'isoler thermiquement le reste de l'objet **2,** à part la pointe. Ainsi, seule la pointe de l'objet **2** est chaude et pas la totalité de l'objet **2.** La peau et les organes pouvant être en contact avec le reste de l'objet **2** ne sont pas chauffés.

La Fig. 10 montre un agrandissement d'un mode de réalisation selon l'invention, dans lequel le dispositif **1** comprenant un objet **2** comprenant une pointe chauffante, pointue, biocompatible, telle qu'une aiguille d'acupuncture et qui comprend au moins un matériau ferromagnétique **9.** La pointe chauffante et pointue est entourée d'un matériau biocompatible **11** tel que du silicone, du polytétrafluoroéthylène (téflon), un polymère hydrophile, et de préférence est du silicone.

La Fig. 11 montre un agrandissement d'un mode de réalisation selon l'invention, dans lequel le dispositif **1** comprenant un objet **2** comprenant une pointe chauffante, pointue, biocompatible, telle qu'une aiguille d'acupuncture et qui comprend au moins un matériau ferromagnétique **9.** Le matériau ferromagnétique **9** est présent dans la totalité de l'objet **2** L'objet **2** est recouvert au moins en partie d'une couche de matériau isolant **10,** de préférence est recouvert totalement d'une couche de matériau isolant **10** à l'exception de la pointe chauffante. Ce matériau isolant peut être choisi parmi tous les matériaux isolants existants, et en particulier peut être choisi parmi les matières plastiques. L'objet **2** est recouvert totalement d'un matériau biocompatible **11** tel que du silicone, du polytétrafluoroéthylène (téflon), un polymère hydrophile, et de préférence le matériau biocompatible est du silicone.

La Fig. 12 montre un dispositif de contrôle de la température **4** du dispositif **1** d'un mode de réalisation selon invention. Dans ce mode de réalisation, le dispositif de contrôle de la température comprend un dispositif d'affichage de la température de la pointe de l'objet **12** et de la température de consigne **15,** des moyens **13** pour déterminer une température de consigne, comme par exemple un bouton + et un bouton -, et un interrupteur **14.** Dans ce mode de réalisation, l'utilisateur peut fixer une température de consigne **14** au moyen des moyens **13.** Une fois cette température de consigne **15** déterminée, la température de la pointe de l'objet, représentée par le dispositif d'affichage **12,** va évoluer jusqu'à atteindre la température de consigne **15.** La température de la pointe de l'objet **2** peut être mesurée par exemple au moyen d'un thermomètre ou d'un thermocouple, non représentés ici, relié au dispositif de contrôle de la température **4.** Pour des raisons de sécurité, l'utilisateur peut garder en permanence le contrôle sur l'élévation de la température de la pointe de l'objet **2** au moyen de l'interrupteur **14** qui permet d'interrompre complétement le chauffage de la pointe de l'objet **2.** Cette interrupteur **14** peut également servir à arrêter à la fin de l'utilisation le dispositif **1** selon l'invention.

Les modes de réalisation présentés dans la suite de la description s'appliquent à l'ensemble de la description et notamment à l'ensemble des modes de réalisation décrit dans les figures de la demande.

### Pointe chauffante

Dans un dispositif permettant de comprendre le contexte de l'invention, la pointe chauffante de l'objet **2** est tranchante, c'est-à-dire qu'elle présente une forme allongée qui présente un coté travaillé de manière à couper de la matière notamment de la peau. Dans un mode de réalisation, la pointe chauffante est une lame de scalpel ou bistouri. Dans un mode de réalisation, l'objet **2** est un scalpel ou bistouri.

Lorsque la pointe chauffante de l'objet **2** est tranchante, dans un dispositif permettant de comprendre le contexte de l'invention, la pointe chauffante peut mesurée de 1 à 40 mm, de préférence de 1 à 20 mm, plus préférentiellement de 3 à 10 mm.

Lorsque la pointe chauffante de l'objet **2** est tranchante, dans un dispositif permettant de comprendre le contexte de l'invention, l'épaisseur de la pointe de l'objet **2** varie d'environ 0,10 mm à 2,0 mm.

Dans un autre mode de réalisation de l'invention, la pointe chauffante de l'objet **2** est pointue, c'est-à-dire qu'elle présente une forme allongée et cylindrique qui est suffisamment fine pour pouvoir transpercer de la matière notamment de la peau : c'est une aiguille d'acupuncture.

Lorsque la pointe chauffante de l'objet **2** est pointue, dans un mode de réalisation, la pointe chauffante peut mesurée de 1 à 40 mm, de préférence de 1 à 20 mm, plus préférentiellement de 3 à 10 mm.

Lorsque la pointe chauffante de l'objet **2** est pointue, dans un mode de réalisation, le diamètre de la pointe de l'objet **2** varie d'environ 0,15 mm à environ 3,3 mm.

La pointe chauffante de l'objet **2** du dispositif **1** selon l'invention comprend au moins un matériau ferromagnétique. Dans un mode de réalisation, le matériau ferromagnétique n'est présent que dans la pointe chauffante de l'objet **2.** Dans un autre mode de réalisation, le matériau ferromagnétique est présent dans tout l'objet **2.**

### Matériau ferromagnétique

Dans un mode de réalisation, le matériau ferromagnétique **9** est choisi parmi les aciers, de préférence choisi parmi les aciers inoxydables ferritiques, les aciers inoxydables martensitiques, et les mélanges de ces aciers,

Dans un mode de réalisation, le matériau ferromagnétique **9** est choisi parmi l'acier inoxydable martensitique 420, l'acier inoxydable martensitique 416, l'acier inoxydable ferritique 430, et les mélanges de ces aciers.

Dans un mode de réalisation, le matériau ferromagnétique **9** est l'acier inoxydable martensitique 420. Ce matériau présente l'avantage d'être à la fois ferromagnétique et biocompatible.

### Matériau biocompatible

Dans un mode de réalisation, la pointe chauffante de l'objet **2** est recouverte d'un matériau biocompatible.

Dans un mode de réalisation, l'objet **2** est recouvert totalement d'un matériau biocompatible.

Dans un mode de réalisation, le matériau biocompatible est de l'acier inoxydable martensitique 420. Dans un mode de réalisation, le matériau ferromagnétique et le matériau biocompatible sont un seul et même composé, l'acier inoxydable martensitique 420.

Dans un autre mode de réalisation, le matériau biocompatible est du silicone, du polytétrafluoroéthylène (téflon) ou un polymère hydrophile, et de préférence est du silicone.

Dans un mode de réalisation, le matériau ferromagnétique est l'acier inoxydable martensitique 416 et le matériau biocompatible est du silicone.

Dans un mode de réalisation, le matériau ferromagnétique est l'acier inoxydable ferritique 430 et le matériau biocompatible est du silicone.

Dans un mode de réalisation, la pointe chauffante de l'objet **2** est recouverte d'une couche d'un matériau biocompatible, par exemple du silicone, d'épaisseur allant de 1 µm à 25 µm, de préférence allant de 1 µm à 10 µm.

### Matériau isolant

Dans un mode de réalisation, l'objet **2** est recouvert au moins en partie d'une couche de matériau isolant **10.** Dans un mode de réalisation, l'objet **2** est recouvert totalement d'une couche de matériau isolant **10** à l'exception de la pointe chauffante. Dans un mode de réalisation, l'objet **2** est recouvert totalement d'une couche de matériau isolant **10** à l'exception d'une zone en contact avec un disque chauffant tel que décrit ci-après, lorsque celui-ci est utilisé. Dans un mode de réalisation, l'objet **2** est recouvert totalement d'une couche de matériau isolant **10** à l'exception de la pointe chauffante et d'une zone en contact avec un disque chauffant tel que décrit ci-après, lorsque celui-ci est utilisé.

Ce matériau isolant peut être choisi parmi tous les matériaux isolants existants, et en particulier peut être choisi parmi les matières plastiques.

Dans un mode de réalisation, l'objet **2** est recouvert totalement d'une couche de matériau isolant **10** à l'exception de la pointe chauffante formant une première couche et l'objet **2** est recouvert totalement d'un matériau biocompatible formant une deuxième couche. Ainsi, au niveau de la pointe chauffante, il y a une seule couche, le matériau biocompatible, alors que pour le reste de l'objet **2,** il y a deux couches, le matériau isolant et le matériau biocompatible, étant entendu que le matériau biocompatible est à l'extérieur de l'objet **2** (la partie en contact avec la peau ou les organes).

Dans un mode de réalisation, l'objet **2** est recouvert totalement d'une couche de matériau isolant **10** à l'exception de la pointe chauffante et d'une zone en contact avec un disque chauffant tel que décrit ci-après, lorsque celui-ci est utilisé, formant une première couche et l'objet **2** est recouvert totalement d'un matériau biocompatible formant une deuxième couche. Ainsi, au niveau de la pointe chauffante et au niveau de la zone en contact avec un disque chauffant tel que décrit ci-après, il y a une seule couche, le matériau biocompatible, alors que pour le reste de l'objet **2,** il y a deux couches, le matériau isolant et le matériau biocompatible, étant entendu que le matériau biocompatible est à l'extérieur de l'objet **2** (la partie en contact avec la peau ou les organes).

### Générateur de champ magnétique

Selon l'invention, le générateur de champ magnétique **3** est disposé autour de l'objet **2.** Selon l'invention, le générateur de champ magnétique **3** à la forme d'un cylindre creux adapté pour entourer l'objet **2.** Selon l'invention, le générateur de champ magnétique **3** à la forme d'un cylindre qui comprend un orifice traversé perpendiculairement par l'objet **2.** Selon l'invention, le générateur de champs magnétique **3** à la forme d'un galet cylindrique destiné à être posé sur la peau **5** qui comprend un orifice traversé perpendiculairement par l'objet **2.**

Dans un mode de réalisation, le générateur de champs magnétique **3** est isolant, c'est-à-dire qu'il est constitué d'une coque en matière isolante. Dans un mode de réalisation, le générateur de champs magnétique **3** est constitué d'une coque en matière plastique. Dans un mode de réalisation, le générateur de champs magnétique **3** est constitué d'une coque en verre. Dans un mode de réalisation, le générateur de champs magnétique **3** est constitué d'une coque en aluminium.

Lorsque le générateur de champs magnétique **3** à la forme d'un galet cylindrique destiné à être posé sur la peau **5,** dans un mode de réalisation, ledit galet est chauffant, c'est-à-dire qu'il est généralement constitué d'une coque en matière conductrice. Dans un mode de réalisation, ledit galet est constitué d'une coque en métal conducteur. Dans un mode de réalisation, le métal conducteur est le même métal que celui de la pointe chauffante de l'objet **2.** Dans un mode de réalisation, le métal conducteur est le même métal que celui de l'objet **2.**

Dans un mode de réalisation, le générateur de champs magnétique **3** n'est pas fixé à l'objet **2.**

Selon l'invention, le générateur de champs magnétique **3** a un diamètre allant de 20 mm à 80 mm. Selon l'invention, le générateur de champs magnétique **3** a une épaisseur allant de 0,5 mm à 25 mm. Dans un mode de réalisation, l'orifice du générateur de champs magnétique **3** a un diamètre allant de 0,1 mm à 10 mm, de préférence de 0,1 mm à 5 mm.

Dans un mode de réalisation, le générateur de champ magnétique **3** comprend une bobine conductrice traversée par un courant alternatif.

Dans un mode de réalisation, la bobine a un diamètre allant de 5 mm à 50 mm, de préférence allant de 5 mm à 30 mm. Dans un mode de réalisation, la bobine a une longueur allant de 10 mm à 100 mm, de préférence allant de 20 mm à 50 mm. Dans un mode de réalisation, la bobine comprend un nombre de spires allant de 50 à 1000, de préférence allant de 100 à 1000.

Dans un mode de réalisation, la bobine est constituée de fil de cuivre.

Dans un mode de réalisation, le générateur de champ magnétique **3** produit un champ magnétique d'intensité allant de 0,01 à 5 T, de préférence de 0,1 à 1 T.

Dans un mode de réalisation, le générateur de champ magnétique **3** comprend plusieurs bobines telles que décrites ci-dessus. Dans un mode de réalisation, le générateur de champ magnétique **3** comprend deux bobines telles que décrites ci-dessus. Dans un mode de réalisation, le générateur de champ magnétique **3** comprend trois bobines telles que décrites ci-dessus. Dans un mode de réalisation, le générateur de champ magnétique **3** comprend quatre bobines telles que décrites ci-dessus. Ces différentes bobines peuvent être commandées séparément par le dispositif de contrôle de la température du dispositif de l'invention ce qui permet, d'une part, d'affiner encore le contrôle de la température, et d'autre part de couvrir une large gamme de températures. Ainsi, ces différentes bobines peuvent être activées séparément en fonction des besoins.

Dans un mode de réalisation, le générateur de champ magnétique peut être activé périodiquement. Ainsi, le générateur de champ magnétique permet de produire des périodes de chauffe et de refroidissement de la pointe de l'objet **2,** par exemple selon un programme cyclique ou encore selon un programme préalablement établi. Dans un mode de réalisation, le générateur de champ magnétique est doté d'un modulateur de fréquence.

Dans un mode de réalisation, le dispositif de contrôle de la température du dispositif de l'invention est relié au générateur de champ magnétique et contrôle le générateur de champ magnétique en fonction de la température de la pointe de l'objet et d'une température de consigne.

Dans un mode de réalisation, le dispositif de contrôle de la température du dispositif de l'invention est relié au générateur de champ magnétique et comprend a) un dispositif d'affichage de la température de la pointe de l'objet, b) des moyens pour déterminer une température de consigne, et c) un dispositif de contrôle du générateur de champ magnétique afin que la température de la pointe de l'objet soit égale à la température de consigne.

Dans un autre mode de réalisation, le dispositif de contrôle de la température du dispositif de l'invention est relié au générateur de champ magnétique et comprend a) un dispositif d'affichage de la température de la pointe de l'objet et un autre dispositif d'affichage de la température au niveau du disque chauffant tel que décrit ci-après, lorsque celui-ci est utilisé, b) des moyens pour déterminer une température de consigne, et c) un dispositif de contrôle du générateur de champ magnétique afin que la température de la pointe de l'objet et la température au niveau du disque chauffant tel que décrit ci-après, lorsque celui-ci est utilisé soient égales à la température de consigne.

### Ferrite

Dans un mode de réalisation, le générateur de champ magnétique **3** comprend en outre de la ferrite **7** disposée de manière à diriger le champ magnétique selon l'axe longitudinal de l'objet **2.**

Dans un mode de réalisation, la ferrite se présente sous la forme d'un cylindre creux adapté pour entourer l'objet **2.**

### Disque

Dans un mode de réalisation, le dispositif **1** comprend en outre un disque **6,** isolant ou chauffant, destiné à être posé sur la peau **5** qui comprend un orifice traversé perpendiculairement par l'objet **2.**

Dans un mode de réalisation, le disque **6** est isolant, c'est-à-dire qu'il est constitué d'une matière isolante. Dans un mode de réalisation, le disque **6** est constitué d'une matière plastique. Dans un mode de réalisation, le disque **6** est constitué de verre.

Dans un autre mode de réalisation le disque **6** est chauffant, c'est-à-dire qu'il est généralement constitué d'une matière conductrice. Dans un mode de réalisation, le disque **6** est constitué de métal conducteur. Dans un mode de réalisation, le métal conducteur est le même métal que celui de la pointe chauffante de l'objet **2.** Dans un mode de réalisation, le métal conducteur est le même métal que celui de l'objet **2.**

Dans un mode de réalisation, le disque **6** a un diamètre allant de 10 mm à 100 mm, de préférence de 20 mm à 80 mm. Dans un mode de réalisation, le disque **6** a une épaisseur allant de 0,1 mm à 5 mm, de préférence de 0,5 mm à 2 mm. Dans un mode de réalisation, l'orifice du disque **6** a un diamètre allant de 0,1 mm à 10 mm, de préférence de 0,1 mm à 5 mm.

### Utilisation cosmétique

Une finalité du dispositif **1** de l'invention est d'être utilisé dans le traitement des ridules, des cernes et de la cellulite superficielle. Dans ce mode de réalisation, le dispositif de contrôle de la température du dispositif de l'invention est configuré pour que la température de la pointe de l'objet **2** soit généralement comprise entre 40°C et 50°C, de préférence comprise entre 40°C et 42°C, et plus préférentiellement soit égale à environ 41,5°C.

### Utilisation thérapeutique

Une finalité du dispositif **1** de l'invention est d'être utilisé dans le traitement des varices. Dans ce cas, le dispositif de contrôle de la température du dispositif de l'invention est configuré pour que la température de la pointe de l'objet **2** soit généralement comprise entre 40°C et 50°C, de préférence comprise entre 40°C et 42°C, et plus préférentiellement soit égale à environ 41,5°C.

Une autre finalité du dispositif **1** de l'invention est d'être utilisé dans le traitement des syndromes reliés à des tableaux de spondylalgies et des douleurs reliées à ces différents tableaux, en particulier des lombalgies ou des cervicalgies chroniques et de leurs douleurs projetées. Dans ce cas, le dispositif de contrôle de la température du dispositif de l'invention est configuré pour que la température de la pointe de l'objet **2** soit généralement comprise entre 75°C et 95°C, de préférence soit comprise entre 80°C et 90°C.

Une autre finalité du dispositif **1** de l'invention est d'être utilisé en remplacement des techniques de radiofréquence (ou nucléoplastie). Dans un cas, le dispositif **1** de l'invention est utilisé en remplacement des techniques de radiofréquence de la jambe. Dans ce cas, le dispositif de contrôle de la température du dispositif de l'invention est configuré pour que la température de la pointe de l'objet **2** soit comprise entre 100°C et 200°C, et de préférence soit comprise entre 100°C et 130°C.

Une autre finalité du dispositif **1** de l'invention est d'être utilisé en alternative des traitements thermiques tel que la nucléolyse à l'oxygène-ozone ou encore l'utilisation d'un laser pour la discectomie percutanée au laser Holmium-Yag. Ces deux techniques sont par exemple décrites dans la thèse de Daniel SPAETER soutenue le 21 Juin 2004 intitulée « Traitement de la sciatique par voie percutanée : intérêt de la nucléoplastie par radiofréquence, à propos de 15 cas » avec le jury composé de : Mr A Gangi, Mr J.L. Dietemann, Mr J.P. Steib, Mr. X. Buy et Mr. P. Laurent.

Une autre finalité du dispositif **1** de l'invention est d'être utilisé dans le traitement de certains cancers, en particulier sur les tumeurs solides de cancers.

Une autre finalité du dispositif **1** de l'invention est d'être utilisé en alternative d'un bistouri électrique. Dans ce cas, la pointe de l'objet chauffant est généralement tranchante.

Bien que divers modes de réalisation aient été décrits et illustrés, la description détaillée ne doit pas être considérée comme étant limitée à ces derniers. Diverses modifications peuvent être apportées aux modes de réalisation par l'homme du métier sans s'écarter du véritable esprit et de la portée de la divulgation telle que définie par les revendications.

### RÉFÉRENCES

- 1 -: Dispositif
- 2 -: Objet comprenant une pointe chauffante
- 3 -: Générateur de champ magnétique
- 4 -: Dispositif de contrôle de la température de la pointe chauffante de l'objet
- 5 -: Peau
- 6 -: Disque
- 7 -: Ferrite
- 8 -: Support
- 9 -: Matériau ferromagnétique
- 10 -: Matériau isolant
- 11 -: Matériau biocompatible
- 12 -: Dispositif d'affichage de la température de la pointe de l'objet
- 13 -: Moyens pour déterminer une température de consigne
- 14 -: Interrupteur
- 15 -: Dispositif d'affichage de la température de consigne

## Revendications

1. Dispositif (1) comprenant :
- un objet (2) comprenant une aiguille d'acupuncture chauffante, pointue et cylindrique, biocompatible, et qui comprend au moins un matériau ferromagnétique (9),
- un générateur de champ magnétique (3) adapté pour diriger le champ magnétique du matériau ferromagnétique afin de produire une élévation de température dans l'aiguille d'acupuncture chauffante de l'objet (2), et
- un dispositif de contrôle de la température (4) de l'aiguille d'acupuncture de l'objet (2)
**caractérisé en ce que** ledit générateur de champ magnétique (3) a la forme d'un galet cylindrique creux, destiné à être posé sur la peau (5), qui comprend un orifice traversé perpendiculairement par ledit objet (2) ; et
**en ce que** le générateur de champ magnétique (3) a un diamètre allant de 20 mm à 80 mm, et une épaisseur allant de 0,5 mm à 25 mm.

2. Dispositif (1) selon la revendication **1, caractérisé en ce que** le générateur de champ magnétique (3) est constitué d'une coque en matière isolante.

3. Dispositif (1) selon la revendication **1, caractérisé en ce que** le générateur de champ magnétique (3) est constitué d'une coque en matière conductrice.

4. Dispositif (1) selon l'une quelconque des revendications **1** à **3, caractérisé en ce que** ledit orifice du générateur de champ magnétique (3) a un diamètre allant de 0,1 mm à 10 mm.

5. Dispositif (1) selon l'une quelconque des revendications **1** à **4, caractérisé en ce que** le matériau ferromagnétique (9) est présent dans tout l'objet (2).

6. Dispositif (1) selon l'une quelconque des revendications **1** à **5, caractérisé en ce que** l'objet (2) est recouvert au moins en partie d'une couche de matériau isolant (10).

7. Dispositif (1) selon l'une quelconque des revendications **1** à **6, caractérisé en ce qu'**il comprend en outre un disque (6), isolant ou chauffant, destiné à être posé sur la peau (5) qui comprend un orifice traversé perpendiculairement par ledit objet.

8. Dispositif (1) selon l'une quelconque des revendications **1** à **7, caractérisé en ce que** ledit générateur de champ magnétique comprend en outre de la ferrite disposée de manière à diriger le champ magnétique selon l'axe longitudinal dudit objet.

9. Dispositif (1) selon l'une quelconque des revendications **1** à **8, caractérisé en ce que** ledit dispositif de contrôle de la température (4) est relié au générateur de champ magnétique (3) et comprend :
a. un dispositif d'affichage (12) de la température de l' aiguille d'acupuncture de l'objet (2),
b. des moyens (13) pour déterminer une température de consigne, et
c. un dispositif de contrôle du générateur de champ magnétique afin que la température de l' aiguille d'acupuncture de l'objet soit égale à la température de consigne.

10. Dispositif (1) selon l'une quelconque des revendications **1** à **9, caractérisé en ce que** ledit matériau ferromagnétique (9) est choisi parmi les aciers et les mélanges de ces aciers.

11. Dispositif (1) selon l'une quelconque des revendications **1** à **10, caractérisé en ce que** ladite aiguille d'acupuncture de l'objet (2) est recouverte au moins en partie d'un matériau biocompatible (11).

## Patentansprüche

1. Vorrichtung (1), umfassend:
- ein Objekt (2), das eine spitze, zylindrische, biokompatible, erhitzbare Akupunkturnadel umfasst, und das mindestens ein ferromagnetisches Material (9) umfasst,
- einen Magnetfeldgenerator (3), der geeignet ist, das Magnetfeld des ferromagnetischen Materials zu lenken, um eine Temperaturerhöhung in der erhitzbaren Akupunkturnadel des Objekts (2) zu erzeugen, und
- eine Temperaturkontrollvorrichtung (4) der Akupunkturnadel des Objekts (2),
**dadurch gekennzeichnet, dass** der Magnetfeldgenerator (3) die Form eines Rades aufweist, die zum Auflegen auf die Haut (5) bestimmt ist, die eine Öffnung aufweist, die senkrecht durch das Objekt (2) hindurchgeht; und
dadurch, dass der Magnetfeldgenerator (3) einen Durchmesser von 20 mm bis 80 mm und eine Dicke von 0,5 mm bis 25 mm aufweist.

2. Vorrichtung (1) nach Anspruch **1, dadurch gekennzeichnet, dass** der Magnetfeldgenerator (3) aus einer Schale aus isolierendem Material besteht.

3. Vorrichtung (1) nach Anspruch **1, dadurch gekennzeichnet, dass** der Magnetfeldgenerator (3) aus einer Schale aus leitfähigem Material besteht.

4. Vorrichtung (1) nach einem der Ansprüche **1** bis **3, dadurch gekennzeichnet, dass** die Öffnung des Magnetfeldgenerators (3) einen Durchmesser von 0,1 mm bis 10 mm aufweist.

5. Vorrichtung (1) nach einem der Ansprüche **1** bis **4, dadurch gekennzeichnet, dass** das ferromagnetische Material (9) im gesamten Objekt (2) vorhanden ist.

6. Vorrichtung (1) nach einem der Ansprüche **1** bis **5, dadurch gekennzeichnet, dass** das Objekt (2) mindestens teilweise mit einer Schicht aus Isoliermaterial (10) bedeckt ist.

7. Vorrichtung (1) nach einem der Ansprüche **1** bis **6, dadurch gekennzeichnet, dass** sie ferner eine isolierende oder erhitzbare Scheibe (6), die zum Auflegen auf die Haut (5) bestimmt ist, umfasst, die eine Öffnung umfasst, die senkrecht durch das Objekt hindurchgeht.

8. Vorrichtung (1) nach einem der Ansprüche **1** bis **7, dadurch gekennzeichnet, dass** der Magnetfeldgenerator angeordnetes Ferrit umfasst, um das Magnetfeld entlang der Längsachse des Objekts zu lenken.

9. Vorrichtung (1) nach einem der Ansprüche **1** bis **8, dadurch gekennzeichnet, dass** die Temperaturkontrollvorrichtung (4) mit dem Magnetfeldgenerator (3) verbunden ist und Folgendes umfasst:
a. eine Vorrichtung (12) zur Anzeige der Temperatur der Akupunkturnadel des Objekts (2),
b. Mittel (13) zur Bestimmung einer Solltemperatur, und
c. eine Vorrichtung zur Steuerung des Magnetfeldgenerators, damit die Temperatur einer Akupunkturnadel des Objekts der Solltemperatur entspricht.

10. Vorrichtung (1) nach einem der Ansprüche **1** bis **9, dadurch gekennzeichnet, dass** das ferromagnetische Material (9) aus Stählen und Mischungen dieser Stähle ausgewählt ist.

11. Vorrichtung (1) nach einem der Ansprüche **1** bis **10, dadurch gekennzeichnet, dass** die Akupunkturnadel des Objekts (2) mindestens teilweise mit einem biokompatiblen Material (11) bedeckt ist.

## Claims

1. Device (1) comprising:
- an object (2) comprising a heating acupuncture needle, pointed and cylindrical, biocompatible, and which comprises at least one ferromagnetic material (9)
- a magnetic field generator (3) suitable for directing the magnetic field of the ferromagnetic material in order to produce an increase in the temperature in the heating acupuncture needle of the object (2), and
- a device for controlling the temperature (4) of the heating acupuncture needle of the object (2)
**characterised in that** said magnetic field generator (3) has a shape of a hollow cylindrical pebble, intended to be placed on the skin (5), which comprises an orifice passed through perpendicularly by said object (2); and
**in that** the magnetic field generator (3) has a diameter ranging from 20 mm to 80 mm, and a thickness ranging from 0.5 mm to 25 mm.

2. Device (1) according to claim **1, characterised in that** the magnetic field generator (3) is formed from a shell made of insulating material.

3. Device (1) according to claim **1, characterised in that** the magnetic field generator (3) is formed from a shell made of a conductive material.

4. Device (1) according to any one of claims **1** to **3, characterised in that** said orifice of the magnetic field generator (3) has a diameter ranging from 0.1 mm to 10 mm.

5. Device (1) according to any one of claims **1** to **4, characterised in that** the ferromagnetic material (9) is present in the entire object (2).

6. Device (1) according to any one of claims **1** to **5, characterised in that** the object (2) is covered at least partially with a layer of insulating material (10).

7. Device (1) according to any one of claims **1** to **6, characterised in that** it further comprises a disc (6), insulating or heating, intended to be placed on the skin (5) which comprises an orifice passed through perpendicularly by said object.

8. Device (1) according to any one of claims **1** to **7, characterised in that** said magnetic field generator further comprises ferrite arranged in such a way as to direct the magnetic field along the longitudinal axis of said object.

9. Device (1) according to any one of claims **1** to **8, characterised in that** said device for controlling the temperature (4) is connected to the magnetic field generator (3) and comprises:
a. a device for displaying (12) the temperature of the acupuncture needle of the object (2)
b. means (13) for determining a setpoint temperature, and
c. a device for controlling the magnetic field generator so that the temperature of the acupuncture needle of the object is equal to the setpoint temperature.

10. Device (1) according to any one of claims **1** to **9, characterised in that** said ferromagnetic material (9) is chosen from steels, and the mixtures of these steels.

11. Device (1) according to any one of claims **1** to **10, characterised in that** said acupuncture needle of the object (2) is covered at least partially with a biocompatible material (11).
